(19) Europäisches Patentamt / European Patent Office / Office européen des brevets

(11) **EP 4 730 337 A1**

(12) **EUROPEAN PATENT APPLICATION**
published in accordance with Art. 153(4) EPC

(43) Date of publication:
**22.04.2026 Bulletin 2026/17**

(21) Application number: **23946363.1**

(22) Date of filing: **15.09.2023**

(51) International Patent Classification (IPC):
***G16B 10/00*** (2019.01)

(52) Cooperative Patent Classification (CPC):
Y02D 30/70

(86) International application number:
**PCT/CN2023/119136**

(87) International publication number:
**WO 2025/020282 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.07.2023 CN 202310921393**

(71) Applicants:
• **The Chinese University of Hong Kong, Shenzhen Shenzhen, Guangdong 518116 (CN)**

• **The Chinese University of Hong Kong, Shenzhen Futian Biomedical Innovation R & D Center Shenzhen, Guangdong 518000 (CN)**

(72) Inventors:
• **ZHU, Lizhe**
  **Shenzhen, Guangdong 518116 (CN)**
• **TI, Rujuan**
  **Shenzhen, Guangdong 518116 (CN)**

(74) Representative: **Metida**
**Gyneju str. 16**
**01109 Vilnius (LT)**

(54) **METHOD AND SYSTEM FOR PREDICTING GPCR ACTIVATION ABILITY OF AGONIST MOLECULES**

(57)    Provided are a method and system for predicting an activation potency of agonist molecules on G Protein-Coupled Receptors (GPCRs). The method includes: blindly speculating complex structures formed by binding of a ligand to an activated receptor structure and an inactivated receptor structure, respectively, via global molecular docking; extracting an initial path enabling an inactivated complex structure to be activated to an activated complex structure based on an enhanced sampling algorithm; searching for a minimum free energy path closest to the initial path by applying an automatic path optimization algorithm; calculating a free energy distribution curve along the minimum free energy path by employing umbrella sampling and determining an energy barrier height and a free energy difference before and after activation, thereby determining the activation potency of the ligand structure on the GPCRs.

FIG. 3

## Description

## TECHNICAL FIELD

The present disclosure relates to computer simulation of biomolecular systems, and in particular to, a method and system for predicting the activation potency of agonist molecules on G Protein-Coupled Receptors (GPCRs).

## BACKGROUND

G Protein-Coupled Receptors (GPCRs) are the biggest membrane protein superfamily (containing 800 members) in human genome encoding. As the most widely distributed intramembrane receptors, GPCRs can be activated by many ligands such as odors, pheromones, hormones, neurotransmitters and chemokines, and subsequently exerts an important influence on almost all aspects of human physiological metabolism through complex signal transduction and regulation mechanisms, playing an important role in various diseases such as cardiovascular diseases, neurological diseases, inflammations, metabolic diseases and cancers. Among the globally approved drugs, more than 30% of the drugs take proteins related to GPCR-associated signaling pathways as therapeutic targets.

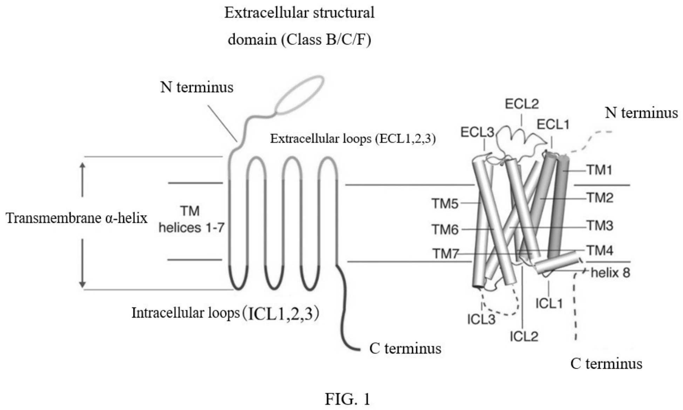

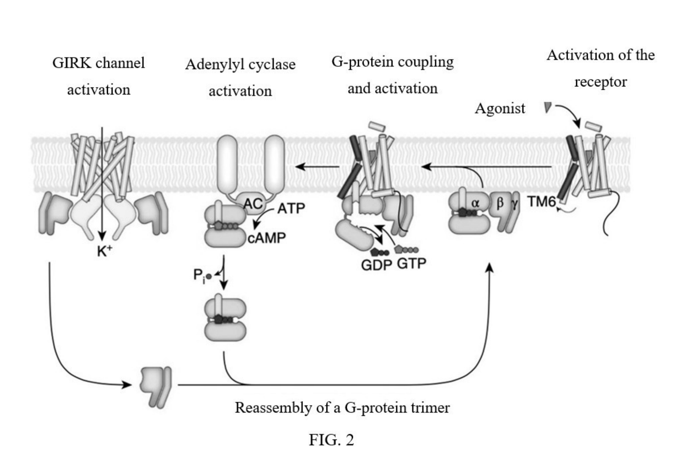

GPCR is composed of seven transmembrane $\alpha$-helixes (TM1-7), three extracellular loops (ECL), and three intracellular loops (ICL), as shown in FIG. 1. After the extracellular end (generally composed of the extracellular loop and the extracellular end of the transmembrane domain in combination) binds to the ligand, the GPCR will undergo a conformational change and switch to an activated state. Subsequently, the binding strength between the intracellular end and a downstream G protein changes, inducing the G protein to exhibit the characteristic of a guanine nucleotide exchange factor. This characteristic facilitates the exchange of guanosine diphosphate (GDP) originally bound to the G protein with guanosine triphosphate (GTP) to further promote the dissociation of an $\alpha$-subunit of the G protein from a $\beta$-subunit and a $\gamma$-subunit, and each of the subunits activates the further downstream signaling pathways. The signaling pathways such as adenylyl cyclase and a potassium ion channel (GIRK) are shown in FIG. 2.

Research on GPCRs has long been a hotspot in both academic circles and pharmaceutical enterprises. After nearly 20 years of efforts, structural biologists have roughly outlined several key states during the activation process of GPCRs and successfully resolved the high-resolution structures of various GPCRs in both activated and inactivated states. The numerous known structures have greatly advanced the design and development of GPCR-targeted drugs. By comparing the resolved structures of class A GPCRs (such as Rhodopsin, $\beta$2AR) in both inactivated and activated states, people have summarized the main differences between GPCRs before and after activation. Although the types of GPCR ligands are highly diverse, these changes are generally regarded as the common characteristics of GPCR activation.

A large number of subtle differences exist in the activation processes of corresponding GPCRs induced by different agonists, and these subtle differences precisely determine the specific (selective) activation of GPCRs by GPCR agonists. As far as drug design targeting GPCRs is concerned, ligand specificity is an unavoidable core issue. Ligand specificity is directly related to its ability to activate a specific target GPCR while failing to activate other GPCR subtypes, and is closely associated with issues such as its activity and toxicity. Ligand specificity is often rooted in the structural and kinetic details of its interaction with GPCRs. In fact, the activation of distinct subtypes within the same GPCR family can be induced by subtle differences among different ligands.

To achieve rational design of the GPCR agonist, it is necessary to predict the specific activation potency of a ligand toward a specific GPCR, while having means to obtain a detailed understanding of the specific mechanism underlying the entire process of ligand binding to the GPCR and subsequent GPCR activation. Only with a mechanistic understanding of the activation process can one obtain the necessary information for optimizing and improving the structure of agonist molecules.

However, it is difficult to meet this demand by current technologies. Functional assays can only be used to determine whether activation occurs. Although structural biology can provide atomic-level details, it only reveals a limited number of states during the process and fails to indicate the difficulty level of activation occurrence as well as its mechanistic origin. Although mutating the sites surrounding a protein pocket one by one to identify key protein residues that interact with ligands is effective, it is excessively costly and cumbersome.

Moreover, main technologies capable of predicting contemporary computer-aided drug design (CADD), such as molecular docking, free energy perturbation (FEP), emphasize on calculation of the ligand binding strength and does not involve the activation process of the receptor after the ligand binds to the target receptor.

Therefore, developing algorithmic tools capable of efficiently revealing the activation mechanism and activation potency of candidate agonists on GPCRs is not only an inherent requirement for studying the activation specificity of ligands, but also an indispensable step for the in-depth development of CADD. There is no algorithm framework capable of quickly and accurately predicting the activation potency of any ligand on any given GPCR, such that the rational design of GPCR-targeted highly specific agonists remains an arduous task.

## SUMMARY

In view of this, the present disclosure provides a method and system for predicting an activation potency of agonist molecules on G Protein-Coupled Receptors (GPCRs). A specific solution is as follows:

A method for predicting an activation potency of agonist molecules on GGPCRs, including:

acquiring, by an agonist, an initial ligand structure, acquiring an inactivated receptor structure and an activated receptor structure of a given GPCR, and determining whether a complex structure formed by binding of the ligand structure to the GPCR is known;

if the complex structure is unknown, blindly speculating an activated complex structure and an inactivated complex structure formed by binding of the ligand structure to the activated receptor structure and the inactivated receptor structure, respectively, by means of a global molecular docking technology, and performing local structural optimization;

extracting a path enabling the inactivated complex structure to be activated to the activated complex structure based on an enhanced sampling algorithm to obtain an initial path of an activation process;

searching for a minimum free energy path closest to the initial path by applying a preset automatic path optimization algorithm; and

calculating a free energy distribution curve along the minimum free energy path by employing umbrella sampling and determining an energy barrier height and a free energy difference before and after activation by means of the free energy distribution curve, thereby determining the activation potency of the ligand structure on the GPCR.

In a specific example, a part of structural information of the minimum free energy path is applied to completing optimized modification of the ligand structure, so as to increase or decrease the capacity of the agonist molecules to activate the given GPCR.

In a specific example, the preset automatic path optimization algorithm includes: travelling-salesman based automated path searching (TAPS), a finite temperature string method, a fast tomographic method, and transition path sampling.

In a specific example, the preset enhanced sampling algorithm includes: a coarse-grained model, a high-temperature simulation algorithm, a steered molecular dynamics algorithm, a targeted molecular dynamics algorithm, and a data-driven path algorithm.

In a specific example, the TAPS specifically includes:

defining a root mean square distance (RMSD) between any two structures by means of all heavy atoms of the complex structure to further calculate a path collective variable (PCV), and projecting a high-dimensional space onto a temporary coordinate system constructed based on a current initial path by means of the PCV;

quickly searching for minimum free energy path segments near the initial path by means of orthogonal direction sampling;

establishing a virtual node with a zero distance from a known path node, performing traveling salesman problem solution on the path nodes of all minimum free energy path segments comprising the virtual node, and after removing the virtual node, completing sorting to obtain a new initial path;

correcting a spatial resolution of the new initial path to complete one iteration; and

when a variation amplitude of the obtained initial path is lower than a preset amplitude, stopping iteration, and taking the initial path as the minimum free energy path.

In a specific example, for a molecular docking structure of the activated complex and the inactivated complex obtained by blind speculating, local structural optimization is performed using short-duration molecular dynamics simulation to obtain an accurate two-state complex structure model.

In a specific example, an external force is applied to all heavy atoms of the inactivated complex structure to pull the inactivated complex structure to the activated complex structure model, so as to generate the initial path of the activation process.

In a specific example, the inactivated receptor structure and the activated receptor structure of the given GPCR are obtained by means of the known structure, homology modeling or AlphaFold.

In a specific example, if the activation potency of the current ligand structure on the GPCR fails to meet preset requirements, optimization and modification are performed on the ligand structure based on complete structures in a transition state and an intermediate metastable state of the minimum free energy path to obtain a new ligand structure, so as to predict the activation potency of the new ligand structure on the GPCR again.

A system for predicting an activation potency of agonist molecules on GPCRs, including the following modules:

a preparation module, configured to acquire, by an agonist, an initial ligand structure, acquire an inactivated receptor structure and an activated receptor structure of a given GPCR, and determine whether a complex structure formed by binding of the ligand structure to the GPCR is known;

a blind-speculating module, configured to, if the complex structure is unknown, blindly speculate an activated complex structure and an inactivated complex structure formed by binding of the ligand structure to the activated receptor structure and the inactivated receptor structure, respectively, by means of a global molecular docking technology, and perform local structural optimization;

an initial path module, configured to extract a path enabling the inactivated complex structure to be activated to the activated complex structure based on an enhanced sampling algorithm to obtain an initial path of an activation process;

a free energy path module, configured to search for a minimum free energy path closest to the initial path by applying a preset automatic path optimization algorithm; and

an activation potency prediction module, configured to calculate a free energy distribution curve along the minimum free energy path by employing umbrella sampling and determine an energy barrier height and a free energy difference before and after activation by means of the free energy distribution curve, thereby determining the activation potency of the ligand structure on the GPCR.

The present disclosure has the following beneficial effects: The method and system for predicting an activation potency of agonist molecules on GPCRs provided by the present disclosure doesnot input information during the entire process on the premise that the ligand structure is unknown, has high prediction universality, and efficiently reveals the activation mechanism and activation potency of candidate agonists on the GPCRs. In the process of blindly speculating the complex structures formed by binding of the ligand to the activated receptor structure and the inactivated receptor structure of the GPCR, all heavy atoms of the complex structure are used as inputs during generation of the initial path, path optimization, and calculation of the free energy curve, which avoids the interference of external selection on prediction results. The resulted minimum free energy path (MFEP) is composed of a series of complete structures of complexes, which is sufficient to provide detailed mechanistic information, offer guiding insights for the optimization and improvement of agonist molecules, and facilitate the iteration of rational design thereof.

**BRIEF DESCRIPTION OF THE DRAWINGS**

FIG. 1 is a schematic diagram of composition of a structural domain of a GPCR.

FIG. 2 is a schematic diagram of a GPCR signal transduction process.

FIG. 3 is a flow diagram of a prediction method provided by the present disclosure.

FIG. 4 is a schematic diagram of a free energy surface result along a minimum free energy path provided by the present disclosure.

FIG. 5 is a comparison schematic diagram of three key states along a path provided by the present disclosure.

FIG. 6 is a schematic diagram of a prediction system module provided by the present disclosure.

To illustrate the technical solutions of the examples of the present disclosure more clearly, the accompanying drawings used in the examples will be briefly described below. It should be understood that the following drawings only show certain examples of the present disclosure and therefore shall not be considered as limiting the scope. For those of ordinary skill in the art, other relevant accompanying drawings can be obtained based on these drawings without creative effort.

Reference numerals: 1, preparation module; 2, blind-speculating module; 3, initial path module; 4, free energy path module; 5, activation potency prediction module; 6, ligand structure modification module.

**DETAILED DESCRIPTION OF THE EMBODIMENTS**

Various examples disclosed by the present disclosure will be more comprehensively described below. The present disclosure may be embodied in various forms, and adjustments and modifications may be made thereto. However, it should be understood that there is no intention to limit the various embodiments disclosed in the present disclosure to the specific embodiments disclosed herein; instead, the disclosure of the present disclosure shall be construed as encompassing all modifications, equivalents and/or alternatives falling within the spirit and scope of the various embodiments disclosed in the present disclosure.

All-atom molecular dynamics (MD) simulation is a computational simulation research method based on Newtonian dynamics for investigating the microscopic properties of biomolecules, aiming at simulating the time-dependent structural fluctuation behaviors of biomolecules under thermal perturbations. After years of development, MD simulation has become one of the standard tools for investigating complex biological macromolecules, and its application in the mechanism research of GPCR activation has also become widespread. However, predicting the activation potency of candidate agonist molecules on GPCRs by using conventional MD simulations as the primary approach still faces numerous challenges.

When molecular dynamics (MD) simulations are run on conventional high-performance computing devices, such as GPU clusters and CPU resources in supercomputing centers, the total length of the simulations can typically only reach the nanosecond to microsecond scale. The activation process of GPCRs, however, may need to overcome a relatively high energy barrier and thus occur on a timescale of hundreds of microseconds or even longer. When the activation potency of the candidate agonist molecules is predicted, the height of such an energy barrier is unknown in advance.

To accomplish the rational design of an agonist, it is necessary to assume that both the molecular structure of the agonist molecules and GPCR-binding sites thereof are unknown. Moreover, the resulted MFEP shall consist of a series of complete structures of the complexes; otherwise, the MFEP will be insufficient to provide detailed mechanistic information, offer guidance for the optimization and improvement of the agonist molecules, and support the iteration of rational design thereof. Since the ligand structure is unknown, the activated structure of the complex is also unknown. Although the inactivated structure of the complex can be inferred by means of molecular docking (Docking), the activated structure shall typically be generated by exerting an external force through targeted molecular dynamics (tMD) simulations, starting from an inactivated structural model and using the known activated structure of a GPCR with a relatively high sequence similarity as a template. In this process, the external force is applied to unknown atoms.

When molecular dynamics (MD) simulations are run on conventional high-performance computing devices, such as GPU clusters and CPU resources in supercomputing centers, the total length of the simulations can typically only reach the nanosecond to microsecond scale. The activation process of GPCRs, however, may need to overcome a relatively high energy barrier and thus occur on a timescale of hundreds of microseconds or even longer. When the activation potency of the candidate agonist molecules is predicted, the height of such an energy barrier is unknown in advance.

The terms used in the various examples of the present disclosure are employed solely for the purpose of describing specific examples and are not intended to limit the various examples of the present disclosure. As used herein, the singular forms are intended to include the plural forms as well, unless the context clearly dictates otherwise. Unless otherwise defined, all terms used herein (including technical and scientific terms) shall have the same meaning as commonly understood by those of ordinary skill in the art to which the various embodiments of the present disclosure pertain. The terms (such as those defined in commonly used dictionaries) shall be construed as having the same meaning as that in the context of the relevant technical field, and shall not be interpreted as having idealized or overly formal meaning, unless clearly defined in the various embodiments of the present disclosure.

**Example 1**

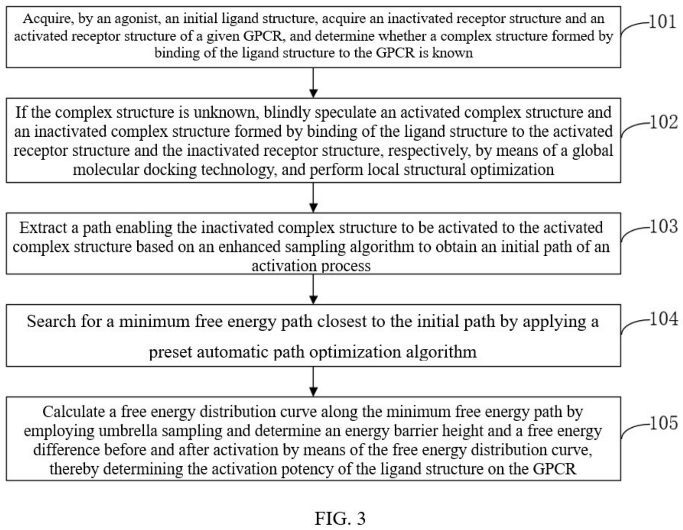

Example 1 of the present disclosure discloses a method for predicting an activation potency of agonist molecules on G Protein-Coupled Receptors (GPCRs), which has not input information during the entire process, has high prediction universality, and efficiently reveals the activation mechanism and activation potency of candidate agonists on the GPCRs. The flow of the method is shown in FIG. 3 in the description, and a specific solution is as follows:

A method for predicting an activation potency of agonist molecules on GPCRs, including the following steps:

101, an agonist acquired an initial ligand structure, acquired an inactivated receptor structure and an activated receptor structure of a given GPCR, and determined whether a complex structure formed by binding of the ligand structure to the GPCR is known;

102, if the complex structure was unknown, an activated complex structure and an inactivated complex structure formed by binding of the ligand structure to the activated receptor structure and the inactivated receptor structure were blindly speculated, respectively, by means of a global molecular docking technology, and local structural optimization was performed;

103, a path enabling the inactivated complex structure to be activated to the activated complex structure was extracted based on an enhanced sampling algorithm to obtain an initial path of an activation process;

104, a minimum free energy path closest to the initial path was searched by applying a preset automatic path optimization algorithm; and

105, a free energy distribution curve along the minimum free energy path was calculated by employing umbrella sampling and an energy barrier height and a free energy difference before and after activation were determined by means of the free energy distribution curve, thereby determining the activation potency of the ligand structure on the GPCR.

In a preferred example, after determining the activation potency of the ligand structure on the given GPCR, the method may further include the following steps:
106, a part of structural information of the minimum free energy path was applied to completing optimized modification of the ligand structure, so as to increase or decrease the capacity of the agonist molecules to activate the given GPCR.

To improve the universality of the ligand structure, the method in this example does not input information in all steps, performs global blind molecular docking, and uses all heavy atoms of the complex structure as inputs during generation of the initial path, path optimization, and calculation of the free energy curve, which reduces the influence of external factors as much as possible.

In this example, the activation potency of the agonist on the given GPCR is predicted, and agonist molecules are used as ligands, and GPCR is used as a receptor. The structure of the receptor in the activated state and the inactivated state can be obtained by means of the known structure, homology modeling or AlphaFold.

The structure of the agonist molecules can be known or unknown (for example, an agonist is given randomly). When the structure of the ligand is unknown (the structure of the agonist molecules is unknown), the inactivated structure of the complex formed by binding of the ligand to the receptor is also unknown. Although the inactivated structure of the complex can be inferred by means of molecular docking, the activated structure shall typically be generated by exerting an external force through tMD simulations, starting from an inactivated structural model and using the known activated structure of a GPCR with a relatively high sequence similarity as a template. In this process, the external force is applied to unknown atoms, which renders subsequent prediction infeasible.

Therefore, in step 101, in addition to acquiring the structure of the receptor in the activated state and the inactivated state, whether the complex structure formed by binding of the ligand to the receptor is known needs to be determined. For example, structural biology research methods such as cryo-electron microscopy and X-ray crystallography can be employed to obtain the complexes formed by the binding of ligands to receptors. When the structures of the complexes are known, there is no need to resort to the global molecular docking technology for blind speculating; instead, the activated complex structures and inactivated complex structures can be obtained directly.

Cryoelectron Microscopy (abbreviated as Cryo-EM) is a method of observing a cryo-fixed sample by employing an electron microscope. The advantage of Cryo-EM lies in its capability to directly observe samples in a liquid or semi-liquid state, such as biological macromolecules and cell membranes. Compared with other structural biology methods, Cryo-EM can obtain high-resolution structures more conveniently.

X-ray crystallography is a method for determining molecular structures by irradiating crystal samples with X-rays. In this method, molecules need to be arranged in a crystal, such that the X-rays can pass through the crystal and generate analyzable diffraction patterns. The advantage of X-ray crystallography lies in its ability to yield high-resolution structures with a resolution of up to 1 Å or even higher.

In actual applications, it is more common that the complex structure formed by the binding of a ligand to a receptor remains unknown. The solution of this example is directed to the scenario where the ligand structure is unknown, and adopts the global molecular docking technology for blind speculating without introducing any input involving external influences. Molecular docking technology performs theoretical simulations based on the characteristics of receptors and the interaction modes between receptors and ligands, investigates intermolecular interactions (for example, those between ligands and receptors), and predicts binding modes and affinities thereof.

The activated complex structure and the inactivated complex structure formed by binding of the ligand structure respectively to the activated receptor structure and the inactivated receptor structure are blindly speculated. For the blindly speculatedactivated complex structure and the inactivated complex structure, the optimal candidates are then selected therefrom for local structural optimization via short-duration molecular dynamics simulation, which improves the precision and reliability of simulation. The short-duration molecular dynamics simulation is a computational biophysics method, which is used to investigate the structures and dynamic properties of biological macromolecules, and simulates the movements of biological macromolecules by means of computer simulation, thereby acquiring information about the structures and dynamic properties of biological macromolecules within a short time frame. In local structural optimization, the short-duration refers to improving the precision and reliability of simulation using a small time step. This helps to avoid errors caused by vibrations or jitters arising from relatively long time steps, thereby rendering the optimization more accurate. Therefore, it is recommended that a relatively short time step be adopted for local structural optimization during molecular dynamics simulation, so as to improve the precision and reliability of the simulation.

The solution of this example is as follows: a preset enhanced sampling algorithm generates an initial path. The initial path is a path where the inactivated complex structure is activated to the activated complex structure. The initial path is generated to better understand the structure and reaction mechanism of molecules, and improve the subsequent simulation efficiency and accuracy as well. The enhanced sampling algorithm is an algorithm for improving the sampling efficiency in molecular dynamics simulation. The preset enhanced sampling algorithm includes: a coarse-grained model, a high-temperature simulation algorithm, a steered MD algorithm, a targeted MD algorithm, and a data-driven path algorithm.

The coarse-grained model is a method that simplifies biological macromolecular structures, which integrates atoms or small molecules in the original molecule into larger components to reduce the computational load and increase the sampling efficiency.

The high-temperature simulation algorithm accelerates the molecular motion by increasing the temperature, so as to increase the sampling efficiency. It is necessary to set parameters such as a simulation temperature, a time step, and a force constant to achieve high-temperature simulation.

The steered MD algorithm controls the motion of the molecule by applying an external force to a certain atom on the molecule, so as to increase the sampling efficiency. It is necessary to set parameters such as a direction of the applied external force, the force constant, the time step to achieve steered MD.

Aiming at a certain structure, the targeted MD algorithm applies an external force to some atoms on the molecule from another structure to drive the atoms to approach the target structure quickly, so as to accelerate sampling. It is necessary to set parameter such as which atoms in the molecule external forces are applied to, the force constant of the applied external force, and the time step to achieve the Steered MD.

The data-driven path algorithm predicts possible reaction paths using machine learning algorithms based on known experimental data or calculation results. It is necessary to prepare datasets for training, select appropriate machine learning models, and adjust hyperparameters of the models to achieve the data-driven path algorithm.

After the initial path is obtained, it is necessary to search for the minimum free energy path. The minimum free energy path in this example is composed of a series of complete complex structures based on a high-dimensional space, rather than a low-dimensional structure in existing path algorithms. Existing algorithms rely on a prior guess of the activation process mechanism, and the resulted path is defined in a low-dimensional coordinate space selected in advance. The complex of the GPCR and the ligand is high in complexity and selection error probability, resulting in a low overall efficiency. The MFEP defined in the low-dimensional space cannot provide enlightenment for further improvement of the ligand structure.

Preferably, a root mean square distance (RMSD) between any two structures is defined by means of all heavy atoms of the complex structure to further calculate a path collective variable (PCV), and by applying the automated path optimization algorithm, started from the initial path, the closest MFEP is found quickly. It is equivalently to define the PCV using all the heavy atoms of the complex. The high-dimensional minimum free energy path of the activation process can be obtained at one time by applying related optimization algorithms, which greatly reduces the computational load, thereby providing a basis for subsequent multiple iterative calculation.

A root mean square deviation (RMSD) is a common method for measuring a deviation of atoms or atomic sets between two structures.

The PCV is defined as follows:

$$s = \frac{\sum_{i=1}^{N} i e^{-\lambda d_{x,i}^2}}{\sum_{i=1}^{N} e^{-\lambda d_{x,i}^2}},$$

$$z = -\frac{1}{\lambda}\ln\left(\sum_{i=0}^{N} e^{-\lambda d_{x,i}^2}\right)$$

where $d_{x,i}$ is an RMSD distance between any conformation in the original high-dimensional space and an $i^{th}$ node of a high-dimensional path (including N high-dimensional node conformations), and $\lambda$ is a constant, decided by a mean value of the RMSDs between adjacent node conformations on the path. $\lambda = 2.3(N-1)/\sum_{i=1}^{N-1} d_{i,i+1}^2$ Through the PCV, the original high-dimensional space can be projected onto a temporary coordinate system equivalent to a current path in this round.

The automated path optimization algorithm includes: travelling-salesman based automated path searching (TAPS), a string method, a fast tomographic method, and transition path sampling. Although specific implementation modes and application scenarios of these algorithms are different, these algorithms aim to find the minimum free energy path by means of optimization or sampling method, i.e., a path that connects an initial state and a target state, such that energy or free energy is minimized.

The TAPS approaches the minimum free energy path gradually by means of multiple iterations. The finite temperature string method represents the structural variation path as a sequence of a series of atom coordinates in the low-dimensional space, and optimizes the same by means of the minimum free energy principle, so as to find the minimum free energy path in the low-dimensional space. The fast tomographic method approaches a true path gradually by iteratively updating the initial path, which is applicable to studying the interactions between small molecules and large molecules. The transition path sampling generates a series of transition state trajectories by means of the Monte Carlo method and then constructs the minimum free energy path, which is applicable to large molecular systems.

Finally, the RMSD and PCV are defined using all the heavy atoms of the complex again, the free energy distribution curve along the MFEP (PCV-s component) is calculated by means of umbrella sampling, and the activation potency of the ligand on the receptor is determined by means of the free energy difference before and after activation and the height of the activation energy barrier.

The free energy curve obtained by umbrella sampling can determine the height of the energy barrier and the free energy difference before and after activation, thereby predicting key information of the activation potency of the ligand on the GPCR. It can be mutually corroborated with the results of GPCR functional experiments (specifically, the bioluminescence resonance energy transfer (BRET) experiment for measuring the dissociation process of downstream G proteins from GPCRs).

The free energy curve can be calculated by means of MetaDynamics. MetaDynamics is a method for calculating the free energy curve, which calculates the free energy using a strategy termed "evolutionary dynamics" and is usually used to calculate the free energy of proteins or other biological molecules to so as to understand their behavior in biological environments. In MetaDynamics, the system will be subjected to a small force, and then the free energy is calculated by calculating the evolutionary behavior of the system. Such a method typically involves simulation across multiple time scales, ranging from microscopic to macroscopic scales, as well as systems with multiple degrees of freedom. MetaDynamics can be combined with other methods in use, such as molecular docking, molecular dynamics simulation, and Monte Carlo simulation, so as to obtain a more accurate free energy curve. Such a method can provide information in the fields related to drug design, protein engineering, and biophysics, and contributes to understanding the structure and function of the biological molecules.

The high-dimensional minimum free energy path is composed of a series of complete complex structures, the complete structures in a transition state and an intermediate metastable state can provide a thought to modify the ligand structure to increase or decrease the capacity thereof to activate the given GPCR. If the activation potency of the current ligand structure on the GPCR fails to meet preset requirements, optimization and modification are performed on the ligand structure based on complete structures in a transition state and an intermediate metastable state of the minimum free energy path to obtain a new ligand structure, so as to predict the activation potency of the new ligand structure on the GPCR again. Since the computational load required in this example is low, integrated with a consumer-grade GPU service, it enables multiple rounds of iterative computation and rational design of ligand molecules, which is of groundbreaking significance.

This example provides a method for predicting an activation potency of agonist molecules on GPCRs does not input information during the entire process on the premise that the ligand structure is unknown, has high prediction universality, and efficiently reveals the activation mechanism and activation potency of candidate agonists on the GPCRs. In the process of blindly speculating the complex structures formed by binding of the ligand to the activated receptor structure and the inactivated receptor structure of the GPCR, all heavy atoms of the complex structure are used as inputs during generation of the initial path, path optimization, and calculation of the free energy curve, which avoids the interference of external selection on prediction results. The resulted minimum free energy path (MFEP) is composed of a series of complete structures of complexes, which is sufficient to provide detailed mechanistic information, offer guiding insights for the optimization and improvement of agonist molecules, and facilitate the iteration of rational design thereof.

**Example 2**

Example 2 of the present disclosure discloses a preferred solution of Example 1. The solution is as follows:

A method for predicting an activation potency of agonist molecules on GPCRs, including the following steps:

　　201, the inactivated receptor structure and the activated receptor structure of the given GPCR were obtained by means of AlphaFold or homology modeling, then the complex structure model of the ligand bound to two states of the receptor was blindly speculated, and optimal candidates were then selected therefrom for local structural optimization via short-duration molecular dynamics simulation, which improves the precision and reliability of simulation.

　　202, an external force was applied to all heavy atoms of the inactivated complex structure to pull the inactivated complex structure to the activated complex structure model, so as to generate the initial path of the activation process.

　　203, a root mean square distance (RMSD) between any two structures was defined by means of all heavy atoms of the complex structure to further calculate a path collective variable (PCV), and by applying the automated path optimization algorithm, started from the initial path, the closest MFEP was found quickly.

　　204, the RMSD and PCV were defined using all the heavy atoms of the complex again, the free energy distribution curve along the MFEP (PCV-s component) was calculated by means of umbrella sampling, and the activation potency of the ligand on the receptor was determined by means of the free energy difference before and after activation and the height of the activation energy barrier.

　　205, optimization and modification on the chemical structure of the ligand were completed by applying the structural information in the transition state and the intermediate state on the MFEP, and returning to 201, the activation potency of the new ligand structure was predicted.

The TAPS quickly searches for the minimum free energy path fragment near the initial path, and then sorts the newly obtained nodes again by applying a traveling-salesman problem solver and corrects the spatial resolution of the new path, thereby iterating for multiple rounds until the path no longer changes to a great extent. The search efficiency of the TAPS in a short peptide system is at least 8 times that of other methods. A traveling-salesman problem rearrangement process is as follows: a virtual node with a zero distance from a known path node was established, traveling salesman problem solution was performed on the path nodes of all minimum free energy path segments comprising the virtual node, and after removing the virtual node, sorting was completed.

The solution in this example quickly searches for the MFEP fragments near the path in a direction orthogonal to the current path by means of nonlocal sampling. On the premise that the String Method has predefined the static coordinate space in advance, the computational efficiency of the TAPS in the short peptide system reaches 8 times that of the former. Moreover, since the new path nodes obtained by non-local sampling in the orthogonal direction do not necessarily follow the permutation order in the previous iteration, the traveling-salesman algorithm is introduced in this example to resort the new path nodes. Since the static coordinate system is not introduced into the TAPS, and in each iteration, by means of a computational mode (which atoms are selected for structural alignment, and which atoms are used for RMSD calculation) for defining the spacing between two conformations (RMSD), the PCV is calculated. It is defined as follows:

$$s = \frac{\sum_{i=1}^{N} i e^{-\lambda d_{\boldsymbol{x},i}^2}}{\sum_{i=1}^{N} e^{-\lambda d_{\boldsymbol{x},i}^2}},$$

$$z = -\frac{1}{\lambda} \ln \left( \sum_{i=0}^{N} e^{-\lambda d_{x,i}^2} \right)$$

where $d_{x,i}$ is an RMSD distance between any conformation in the original high-dimensional space and an $i^{th}$ node of a high-dimensional path (including N high-dimensional node conformations), and $\lambda$ is a constant, decided by a mean value of the RMSDs between adjacent node conformations on the path. $\lambda = 2.3(N-1)/\sum_{i=1}^{N-1} d_{i,i+1}^2$

By means of the PCV, the original high-dimensional space can be projected onto the temporary coordinate system based on the current path of the current iteration, thereby circumventing the challenge of selecting the static coordinate system, greatly improving the degree of automation and overall efficiency of path optimization, and successfully elucidating the functional conformational change mechanisms of multiple complex systems at low cost. Unlike other methods that take a prior conjecture about the mechanism of the process under study as an input, this example is based on path variables, directly obtains the high-dimensional minimum free energy path without the need to predefine a physically meaningful coordinate space, maximally decouples the sampling process from the mechanism interpretation, and thus significantly improves the degree of automation of the algorithm.

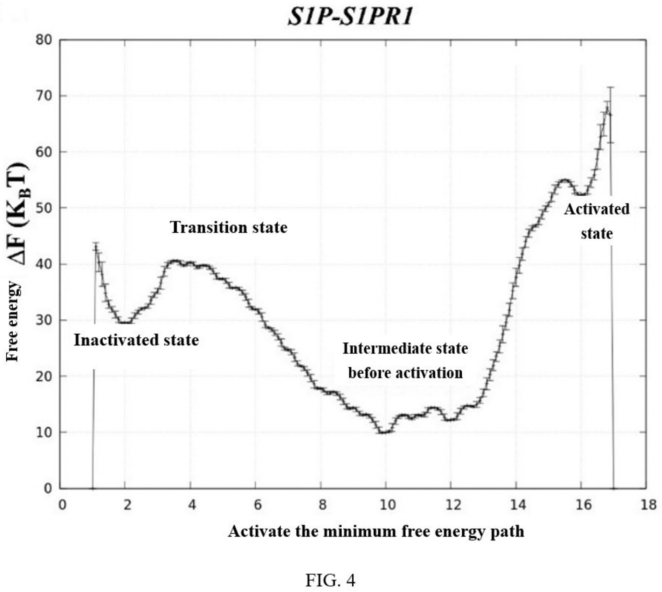

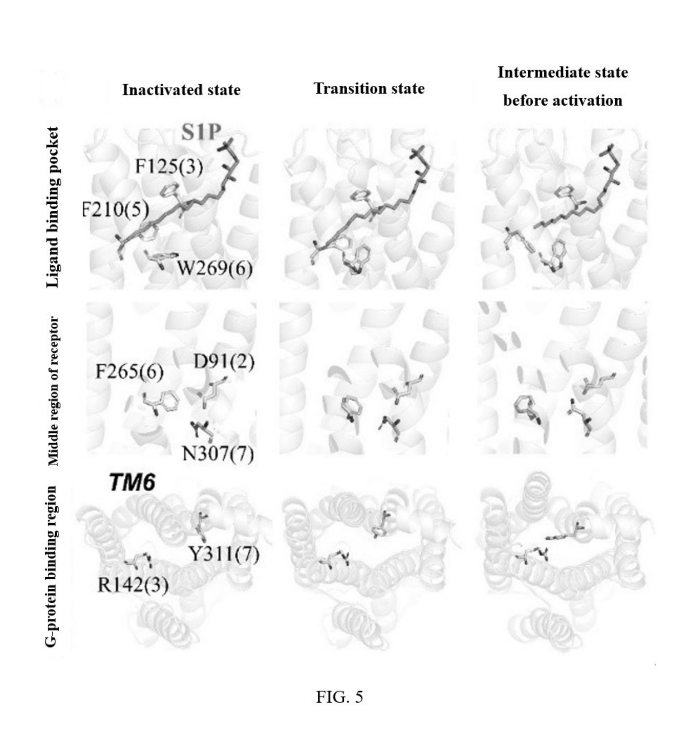

By adopting the solution in this example, the minimum free energy path for the activation of a receptor S1PR1 by sphingosine-1-phosphate (S1P) has been obtained through 12 days of calculation on a server equipped with 8 consumer-grade GPUs (NVIDIA RTX 2080Ti). The minimum free energy path for the activation of S1PR by the ligand S1P is shown in FIGS. 4 and 5. FIG. 4 shows a free energy surface (umbrella sampling) along the resulted minimum free energy path. FIG. 5 shows a comparison of the structures of three key states along the path (the inactivated state, the transition state, and inactivated intermediate state) in the binding pocket, a middle region of the receptor, and an intracellular G protein-binding region, where the key residues that interact with S1P and reflect the major changes during the receptor activation process have all been marked in the inactivated state.

## Example 3

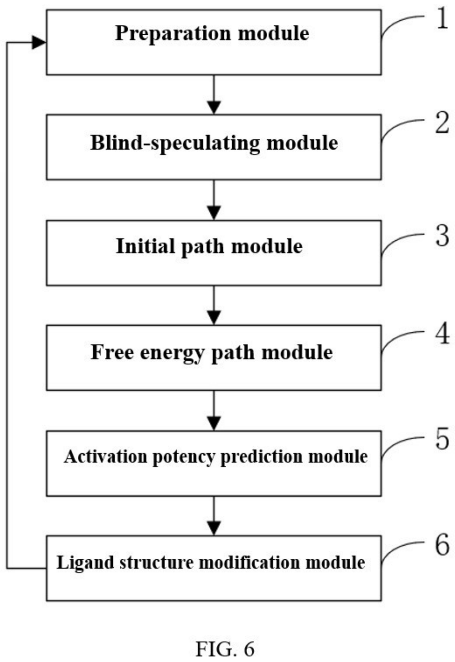

This example provides a system for predicting an activation potency of agonist molecules on G Protein-Coupled Receptors (GPCRs). Based on Example 1, the method in Example 1 is systematized. A specific structure is shown in FIG. 6 in the description, and a specific solution is as follows:

a preparation module 1, configured to acquire, by an agonist, an initial ligand structure, acquire an inactivated receptor structure and an activated receptor structure of a given GPCR, and determine whether a complex structure formed by binding of the ligand structure to the GPCR is known;

a blind-speculating module 2, configured to, if the complex structure is unknown, blindly speculate an activated complex structure and an inactivated complex structure formed by binding of the ligand structure to the activated receptor structure and the inactivated receptor structure, respectively, by means of a global molecular docking technology, and perform local structural optimization;

an initial path module 3, configured to extract a path enabling the inactivated complex structure to be activated to the activated complex structure based on an enhanced sampling algorithm to obtain an initial path of an activation process;

a free energy path module 4, configured to search for a minimum free energy path closest to the initial path by applying a preset automatic path optimization algorithm;

an activation potency prediction module 5, configured to calculate a free energy distribution curve along the minimum free energy path by employing umbrella sampling and determine an energy barrier height and a free energy difference before and after activation by means of the free energy distribution curve, thereby determining the activation potency of the ligand structure on the GPCR; and

a ligand structure modification module 6, configured to optimize and modify the ligand structure by applying a part of structural information of the minimum free energy path, so as to increase or decrease the capacity of the agonist molecules to activate the GPCR.

This example provides a system for predicting an activation potency of agonist molecules on G Protein-Coupled Receptors (GPCRs). The method in Example 1 is systematized, such that the method is of high practicality.

The present disclosure provides a method and system for predicting an activation potency of agonist molecules on GPCRs, which does not input information during the entire process on the premise that the ligand structure is unknown, has high prediction universality, and efficiently reveals the activation mechanism and activation potency of candidate agonists on the GPCRs. In the process of blindly speculating the complex structures formed by binding of the ligand to the activated receptor structure and the inactivated receptor structure of the GPCR, all heavy atoms of the complex structure are used as inputs during generation of the initial path, path optimization, and calculation of the free energy curve, which avoids the interference of external selection on prediction results. The resulted minimum free energy path (MFEP) is composed of a series of complete structures of complexes, which is sufficient to provide detailed mechanistic information, offer guiding insights for the optimization and improvement of agonist molecules, and facilitate the iteration of rational design thereof.

Those skilled in the art will appreciate that the accompanying drawings are merely schematic diagrams of a preferred example, and that the modules or processes shown in the drawings are not necessarily essential to the implementation of the present disclosure. Those skilled in the art will appreciate that the modules in the apparatus according to the example may be distributed within the said apparatus in accordance with the description of the example, or may be correspondingly modified and disposed in one or more apparatuses different from the present example. The modules in the aforementioned example may be combined into a single module or further divided into a plurality of sub-modules. The serial numbers of the present disclosure described above are provided merely for the purpose of description and do not indicate any superiority or inferiority of the respective examples. The foregoing disclosure merely sets forth several specific examples of the present disclosure. However, the present disclosure is not limited thereto, and any variations conceivable to those skilled in the art shall fall within the scope of protection of the present disclosure.

## Claims

1. A method for predicting an activation potency of agonist molecules on G Protein-Coupled Receptors (GPCRs), comprising:

   acquiring, by an agonist, an initial ligand structure, acquiring an inactivated receptor structure and an activated receptor structure of a given GPCR, and determining whether a complex structure formed by binding of the ligand structure to the GPCR is known;
   if the complex structure is unknown, blindly speculating an activated complex structure and an inactivated complex structure formed by binding of the ligand structure to the activated receptor structure and the inactivated receptor structure, respectively, by means of a global molecular docking technology, and performing local structural optimization;
   extracting a path enabling the inactivated complex structure to be activated to the activated complex structure based on an enhanced sampling algorithm to obtain an initial path of an activation process;
   searching for a minimum free energy path closest to the initial path by applying a preset automatic path optimization algorithm; and
   calculating a free energy distribution curve along the minimum free energy path by employing umbrella sampling and determining an energy barrier height and a free energy difference before and after activation by means of the free energy distribution curve, thereby determining the activation potency of the ligand structure on the GPCR.

2. The method according to claim 1, wherein a part of structural information of the minimum free energy path is applied to completing optimized modification of the ligand structure, so as to increase or decrease the capacity of the agonist molecules to activate the given GPCR.

**3.** The method according to claim 1, wherein the preset automatic path optimization algorithm comprises: travelling-salesman based automated path searching (TAPS), a finite temperature string method, a fast tomographic method, and transition path sampling.

**4.** The method according to claim 1, wherein the preset enhanced sampling algorithm comprises: a coarse-grained model, a high-temperature simulation algorithm, a steered molecular dynamics algorithm, a targeted molecular dynamics algorithm, and a data-driven path algorithm.

**5.** The method according to claim 3, wherein the TAPS specifically comprises:

defining a root mean square distance (RMSD) between any two structures by means of all heavy atoms of the complex structure to further calculate a path collective variable (PCV), and projecting a high-dimensional space onto a temporary coordinate system constructed based on a current initial path by means of the PCV;
quickly searching for minimum free energy path segments near the initial path by means of orthogonal direction sampling;
establishing a virtual node with a zero distance from a known path node, performing traveling salesman problem solution on the path nodes of all minimum free energy path segments comprising the virtual node, and after removing the virtual node, completing sorting to obtain a new initial path;
correcting a spatial resolution of the new initial path to complete one iteration; and
when a variation amplitude of the obtained initial path is lower than a preset amplitude, stopping iteration, and taking the initial path as the minimum free energy path.

**6.** The method according to claim 1, wherein for a molecular docking structure of the activated complex and the inactivated complex obtained by blind speculating, local structural optimization is performed using short-duration molecular dynamics simulation to obtain an accurate two-state complex structure model.

**7.** The method according to claim 1, wherein an external force is applied to all heavy atoms of the inactivated complex structure to pull the inactivated complex structure to the activated complex structure model, so as to generate the initial path of the activation process.

**8.** The method according to claim 1, wherein the inactivated receptor structure and the activated receptor structure of the given GPCR are obtained by means of the known structure, homology modeling or AlphaFold.

**9.** The method according to claim 2, wherein if the activation potency of the current ligand structure on the GPCR fails to meet preset requirements, optimization and modification are performed on the ligand structure based on complete structures in a transition state and an intermediate metastable state of the minimum free energy path to obtain a new ligand structure, so as to predict the activation potency of the new ligand structure on the GPCR again.

**10.** A system for predicting an activation potency of agonist molecules on G Protein-Coupled Receptors (GPCRs), comprising the following modules:

a preparation module, configured to acquire, by an agonist, an initial ligand structure, acquire an inactivated receptor structure and an activated receptor structure of a given GPCR, and determine whether a complex structure formed by binding of the ligand structure to the GPCR is known;
a blind-speculating module, configured to, if the complex structure is unknown, blindly speculate an activated complex structure and an inactivated complex structure formed by binding of the ligand structure to the activated receptor structure and the inactivated receptor structure, respectively, by means of a global molecular docking technology, and perform local structural optimization;
an initial path module, configured to extract a path enabling the inactivated complex structure to be activated to the activated complex structure based on an enhanced sampling algorithm to obtain an initial path of an activation process;
a free energy path module, configured to search for a minimum free energy path closest to the initial path by applying a preset automatic path optimization algorithm; and
an activation potency prediction module, configured to calculate a free energy distribution curve along the minimum free energy path by employing umbrella sampling and determine an energy barrier height and a free energy difference before and after activation by means of the free energy distribution curve, thereby determining the activation potency of the ligand structure on the GPCR.

FIG. 1

FIG. 2

EP 4 730 337 A1

| Acquire, by an agonist, an initial ligand structure, acquire an inactivated receptor structure and an activated receptor structure of a given GPCR, and determine whether a complex structure formed by binding of the ligand structure to the GPCR is known | ⌐101 |

↓

| If the complex structure is unknown, blindly speculate an activated complex structure and an inactivated complex structure formed by binding of the ligand structure to the activated receptor structure and the inactivated receptor structure, respectively, by means of a global molecular docking technology, and perform local structural optimization | ⌐102 |

↓

| Extract a path enabling the inactivated complex structure to be activated to the activated complex structure based on an enhanced sampling algorithm to obtain an initial path of an activation process | ⌐103 |

↓

| Search for a minimum free energy path closest to the initial path by applying a preset automatic path optimization algorithm | ⌐104 |

↓

| Calculate a free energy distribution curve along the minimum free energy path by employing umbrella sampling and determine an energy barrier height and a free energy difference before and after activation by means of the free energy distribution curve, thereby determining the activation potency of the ligand structure on the GPCR | ⌐105 |

FIG. 3

14

FIG. 4

FIG. 5

EP 4 730 337 A1

FIG. 6

## INTERNATIONAL SEARCH REPORT

| | International application No. |
|---|---|
| | **PCT/CN2023/119136** |

### A. CLASSIFICATION OF SUBJECT MATTER

G16B 10/00(2019.01)i

According to International Patent Classification (IPC) or to both national classification and IPC

### B. FIELDS SEARCHED

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CNTXT: CNABS; VEN; USTXT; EPTXT; WOTXT; CNKI; IEEE: 激动剂, 配体, 激活, 活性, G蛋白偶联受体, 预测, 结构, 路径, 路线, 自由能, 采样, 分布, 曲线, 分子对接, 复合物, agonist, ligand, activate, activity, GPCR, G Protein-Coupled Receptor, predict, structure, path, route, free energy, sample, distribution, curve, molecular docking, compound

### C. DOCUMENTS CONSIDERED TO BE RELEVANT

| Category* | Citation of document, with indication, where appropriate, of the relevant passages | Relevant to claim No. |
|---|---|---|
| PX | CN 116631495 A (THE CHINESE UNIVERSITY OF HONG KONG, SHENZHEN et al.) 22 August 2023 (2023-08-22) <br> claims 1-10, and description, paragraphs 24-65 | 1-10 |
| A | CN 110400605 A (NANJING UNIVERSITY OF POSTS AND TELECOMMUNICATIONS) 01 November 2019 (2019-11-01) <br> description, paragraphs 30-89 | 1-10 |
| A | CN 115116537 A (THE CHINESE UNIVERSITY OF HONG KONG, SHENZHEN et al.) 27 September 2022 (2022-09-27) <br> description, paragraphs 25-55 | 1-10 |
| A | CN 102930152 A (SHANGHAI INSTITUTE OF MATERIA MEDICA, CHINESE ACADEMY OF SCIENCES et al.) 13 February 2013 (2013-02-13) <br> entire document | 1-10 |
| A | US 2005053999 A1 (GOUGH, David A et al.) 10 March 2005 (2005-03-10) <br> entire document | 1-10 |

☐ Further documents are listed in the continuation of Box C.    ☑ See patent family annex.

| | |
|---|---|
| *   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **18 March 2024** | **26 March 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

International application No.

**PCT/CN2023/119136**

| Patent document cited in search report | | | Publication date (day/month/year) | Patent family member(s) | | | Publication date (day/month/year) |
|---|---|---|---|---|---|---|---|
| CN | 116631495 | A | 22 August 2023 | CN | 116631495 | B | 21 November 2023 |
| CN | 110400605 | A | 01 November 2019 | | None | | |
| CN | 115116537 | A | 27 September 2022 | CN | 115116537 | B | 06 December 2022 |
| CN | 102930152 | A | 13 February 2013 | CN | 102930152 | B | 03 August 2016 |
| US | 2005053999 | A1 | 10 March 2005 | WO | 2006057763 | A2 | 01 June 2006 |
| | | | | WO | 2006057763 | A3 | 16 April 2009 |

Form PCT/ISA/210 (patent family annex) (July 2022)